# EUROPEAN PATENT APPLICATION

(11) **EP 1 649 854 A1**
(43) Date of publication of application: **26.04.2006**
(21) Application number: 04747796.3
(22) Date of filing: 14.07.2004
(51) Int. Cl.: A61K 31/353, A61K 31/352, A61K 9/48, A61K 47/14, A61K 36/18, A61P 3/04, A61P 3/06, A61P 3/10, A61P 9/12, A23L 1/30

(54) **PROCESSED FAT COMPOSITION FOR PREVENTING/AMELIORATING LIFESTYLE-RELATED DISEASES**

(30) Priority: 31.07.2003 JP 2003204275
(71) Applicant: KANEKA CORPORATION, Osaka-shi, Osaka 530-8288 (JP)
(72) Inventor: ARAI, Naoki, Ibaraki-shi, Osaka 5670824 (JP); KISHIDA, Hideyuki, Kakogawa-shi, Hyogo 6750039 (JP); IKEHARA, Toshinori, Takasago-shi, Hyogo 6760011 (JP); MAE, Tatsumasa, Kakogawa-shi, Hyogo 6750111 (JP)
(74) Representative: HOFFMANN EITLE
(86) International application number: PCT/JP2004/010381
(87) International publication number: WO 2005/011672

(57) **Abstract**

The obj ect of the invention is to provide a composition for prevention and/or amelioration of life-style related disease, and/or a composition for inhibition and/or amelioration of increase in body weight, comprising at least one compound selected from the group consisting of glabrene, glabridin, glabrol, 3'-hydroxy-4'-*O*-methylglabridin, 4' -*O*-methylglabridin and hyspaglabridin B as compounds having a PPARγ ligand activity effective in amelioration of insulin resistance and in prevention and/or amelioration of life-style related diseases such as visceral fat obesity, type 2 diabetes, hyperlipemia and hypertension, which can be utilized in food and drink such as health food and health functional food (food for specifieduses and food with nutrient function) , pharmaceutical preparations, non-pharmaceutical preparations, cosmetics etc. At least one compound selected from the group consisting of glabrene, glabridin, glabrol, 3'-hydroxy-4'-*O*-methylglabridin, 4' -*O*-methylglabridin and hyspaglabridin B is dissolved in medium-chain fatty acid triglycerides and/or partial glycerides, whereby the stability of the compound (s) is improved and the processability thereof into food and drink, pharmaceutical preparations, non-pharmaceutical preparations, cosmetics etc. is also improved.

## Description

### Technical Field

The present invention relates to a fat and oil processed composition (processed fat and oil composition, or processed composition containing fats and oils) for prevention and/or amelioration of life-style related disease, and/or a fat and oil processed composition for inhibition and/or amelioration of increase in body weight, which can be utilized for foods and drinks such as health foods, foods with health claims (foods for specified uses [FOSHU] and foods with nutrient function claims), pharmaceutical preparations, non-pharmaceutical preparations, cosmetics etc.

### Background Art

In recent years, the number of persons with obesity is increasing due to deteriorations in the environment of daily life such as westernization of dietary habits and lack of exercise, and life-style related disease accompanying increase in obesity has been regarded as a serious social problem. The life-style related disease includes visceral fat obesity, type 2 diabetes, hyperlipemia, and hypertension, and these diseases belong to the multiple risk factor syndrome finally causing diseases in blood vessels, such as arteriosclerosis. Accordingly, there is demand for a composition having an inhibitory effect on increase in body weight caused by obesity, to prevent and/or ameliorate such life-style related diseases, and as a form of the composition, foods and drinks such as health foods and foods with health claims (foods for specified uses [ FOSHU] and foods with nutrient function claims) capable of daily ingestion, or pharmaceutical preparations, non-pharmaceutical preparations, cosmetics etc. are demanded.

The multiple risk factor syndrome is considered as ' ' the metabolic syndrome' ' in the same morbid concept as that of ' ' syndrome X' ' according to Reaven (Diabetes, 37, 1595-1607, 1988), ' 'the deathly quartet" according to Kaplan (Archives of Internal Medicine, 149, 1514-1520, 1989), " the insulin resistance syndrome" according to DeFronzo (Diabetes Care, 14, 173-194, 1991) and " the visceral fat syndrome" according to Matsuzawa (Diabetes/Metabolism Reviews, 13, 3-13, 1997), and a causative factor common among these syndromes is estimated to be insulin resistance.

It is revealed that as drugs for ameliorating insulin resistance, thiazolidine derivatives such as troglitazone, pioglitazone and rosiglitazone act as ligands for an intranuclear receptor peroxisome proliferator-activated receptor γ (PPARγ), ameliorate insulin resistance in patients with type 2 diabetes, and exhibit a hyperglycemic action. It is also revealed that these thiazolidine derivatives increase subcutaneous fat amounts but decrease visceral fat amounts and exhibit a blood free fatty acid lowering action, a blood pressure lowering action and an anti-inflammatory action (see Martens, F. M., et al., Drugs, 62, 1463-1480, 2002). That is, those compounds having a PPARγ ligand activity are effective in amelioration of insulin resistance and in prevention and/or amelioration of life-style related diseases such as visceral fat obesity, type 2 diabetes, hyperlipemia, and hypertension.

Licorice is a plant of the leguminous *Glycyrrhiza,* and is utilized as foods and medicines (herbal medicines) , and its major species include *Glycyrrhiza glabra, G. uralensis, G. inflata* etc. A hydrophilic component glycyrrhizin (glycyrrhizinic acid) is contained in any species of licorice, while a hydrophobic component flavonoid is contained as a specific compound depending on species. This species-specific flavonoid is sometimes utilized for identification of licorice species.

Among extracts obtained from the licorice, a hydrophobic licorice extract containing a large amount of licorice flavonoids and a very small amount of glycyrrhizin is found to be useful for prevention and/or amelioration of the multiple risk factor syndrome (see WO 02/47699).

On the other hand, the hydrophobic licorice flavonoids contained in the hydrophobic licorice extract are hardly dissolved in water, are easily hardened when being in the form of an organic solvent extract, are rapidly colored to undergo a significant change with time, and are thus difficult to be utilized. For solving this problem, the hydrophobic licorice flavonoids are dissolved in medium-chain fatty acid triglycerides, and the resulting hydrophobic licorice flavonoid preparation is used as an antioxidant, an antibacterial agent, an enzyme inhibitor, a colorant, an antitumor agent, an anti-allergic agent and an antiviral agent (see Japanese Patent No. 2794433). In Japanese Patent No. 2794433, however, the compounds are not limited, and their use as an agent for prevention and/or amelioration of life-style related diseases and their action on inhibition and/or amelioration of increase in body weight are not known.

### Problem to Be Solved by the Invention

In view of the foregoing, glabrene, glabridin, glabrol, 3'-hydroxy-4'-*O*-methylglabridin, 4'-*O*-methylglabridin and hyspaglabridin B have a PPARγ ligand activity, and are thus effective in amelioration of insulin resistance and in prevention and/or amelioration of life-style related diseases such as visceral fat obesity, type 2 diabetes, hyperlipemia, and hypertension. However, these compounds are poor in water solubility and stability, and are thus disadvantageous in difficult application to foods, drinks and pharmaceutical preparations. Accordingly, the objective of the present invention is to provide a composition for prevention and/or amelioration of life-style related diseases, and/or a composition for inhibition and/or amelioration of increase in body weight, comprising at least one compound selected from the group consisting of glabrene, glabridin, glabrol, 3'-hydroxy-4'-*O*-methylglabridin, 4'-*O*-methylglabridin and hyspaglabridin B, which can be utilized in a food or a drink such as a health food, a food with health claims (a food for specified health uses [ FOSHU] , a food with nutrient function claims) , or a pharmaceutical preparation, a quasi drug, or a cosmetic etc.

### Summary of Invention

The present inventors made extensive study in view of the circumstances described above, and as a result, they found that glabrene, glabridin, glabrol, 3'-hydroxy-4'-*O*-methylglabridin, 4' -*O*-methylglabridin and hyspaglabridin B or a salt, an ester or a glycoside thereof are dissolved specifically in certain kinds of fats and oils thereby improving the stability of these compounds, also improving the processability thereof into food and drink, pharmaceutical preparations, non-pharmaceutical preparations, cosmetics etc., and exhibiting an action on inhibition of increase in body weight under excessive nutrition, and the present invention was thereby completed.

That is, the first aspect of the invention relates to a fat and oil processed composition for prevention and/or amelioration of life-style related disease, which contains fats and oils having at least one compound selected from the group consisting of glabrene, glabridin, glabrol, 3'-hydroxy-4' -*O*-methylglabridin, 4' -*O*-methylglabridin and hyspaglabridin B or a salt, an ester or a glycoside thereof dissolved therein, and the second aspect of the invention relates to a fat and oil processed composition for inhibition and/or amelioration of increase in body weight, which contains fats and oils having at least one compound selected from the group consisting of glabrene, glabridin, glabrol, 3'-hydroxy-4'-*O*-methylglabridin, 4'-*O*-methylglabridin and hyspaglabridin B or a salt, an ester or a glycoside thereof dissolved therein.

### Best Mode for Carrying Out the Invention

Hereinafter, the present invention is described in detail by reference to embodiments thereof.

The fat and oil processed composition for prevention and/or amelioration of life-style related diseases and the fat and oil processed composition for inhibition and/or amelioration of increase in body weight according to the present invention comprise fats and oils having at least one compound selected from the group consisting of glabrene, glabridin, glabrol, 3'-hydroxy-4' -*O*-methylglabridin, 4'-*O*-methylglabridin and hyspaglabridin B or a salt, an ester or a glycoside thereof dissolved therein. As used herein, the fat and oil processed composition is fats and oils having the compound(s) dissolved therein or a composition for food and drink, pharmaceutical preparations, non-pharmaceutical preparations, cosmetics etc., containing the fats and oils. That is, the fat and oil processed composition includes a composition having the compounds dissolved in fats and oils, a composition comprising another object mixed with the above composition, and a composition for foods and drinks, pharmaceutical preparations, non-pharmaceutical preparations, cosmetics etc., which is prepared by processing the above composition. Preferably, the composition contains glabridin and at least one compound selected from the group consisting of glabrene, glabrol, 3'-hydroxy-4' -*O*-methylglabridin, 4' *-O*-methylglabridin and hyspaglabridin B. The composition more preferably contains glabridin, glabrene, and at least one compound selected from the group consisting of glabrol, 3' -hydroxy-4' -*O*-methylglabridin, 4' -*O*-methylglabridin and hyspaglabridin B. The composition comprises most preferably all of glabrene, glabridin, glabrol, 3'-hydroxy-4'-*O*-methylglabridin, 4' -*O*-methylglabridin and hyspaglabridin B. The total amount of the compounds is preferably about 0.01 to 30% by weight, more preferably about 0.1 to 30% by weight, still more preferably about 1 to 10% by weight, based on the fat and oil processed composition. When the total amount is higher than about 30% by weight, the compounds may not be dissolved in fats and oils. When the total amount is lower than 0.1% by weight, the effect of the invention may not be sufficiently exhibited. The compounds, like thiazolidinedione derivatives such as troglitazone and pioglitazone, have a PPARγ ligand activity and are thus effective in amelioration of insulin resistance and in prevention and/or amelioration of life-style related diseases such as visceral fat obesity, type 2 diabetes, hyperlipemia and hypertension.

Glabrene, glabridin, glabrol, 3'-hydroxy-4'-*O*-methylglabridin, 4' -*O*-methylglabridin and hyspaglabridin B contained in the fat and oil processed composition of the present invention are effective in inhibition and/or amelioration of increase in body weight caused by excessive nutrition such as ingestion of high fat/high sugar food.

Glabridin, 3' -hydroxy-4' -*O*-methylglabridin, 4' -*O*-methylglabridin and hyspaglabridin B used in the present invention are flavonoids classified into isoflavans, and are compounds representable by the following general formula (1): 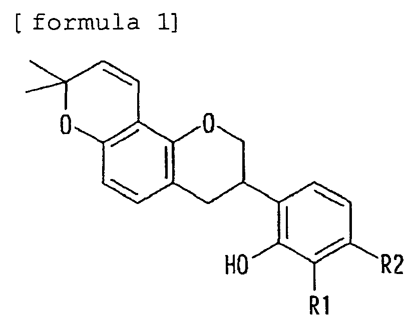 wherein in glabridin, R1 = H, R2 =OH; in 3' -hydroxy-4' -*O*-methylglabridin, R1 = OH, R2 = OCH₃; in 4' -*O*-methylglabridin, R1 = H, R2 = OCH₃; and in hyspaglabridin B, R1 and R2 together are -CH=CH-C (CH₃)₂-O- to constitute a 6-memberred ring.

Glabrene used in the present invention is a flavonoid classified into isoflav-3-ene, and is a compound representable by the following formula (2):

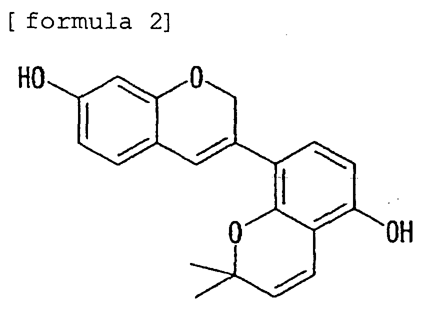

Glabrol used in the present invention is a flavonoid classified into flavanones, and is a compound representable by the following formula (3) :

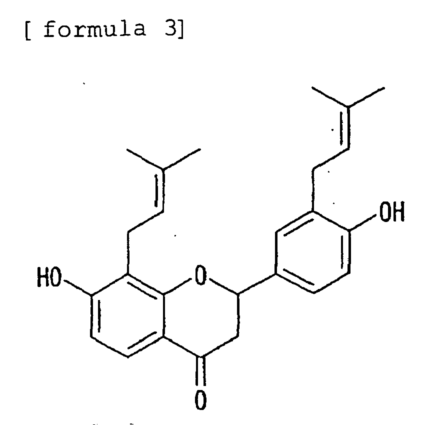

Glabrene, glabridin, glabrol, 3'-hydroxy-4'-*O*-methylglabridin, 4'-O-methylglabridin and hyspaglabridin B are components contained specifically in *Glycyrrhiza glabra* among licorice, and are hardly contained in other species. However, these compounds may be contained in hybrids between G. *glabra* and another species. The compounds can be separated from other flavonoid components, detected and quantified by HPLC analysis using a reverse phase column such as ODS.

The fatty acid esters of the above compounds may also be used in the present invention. The ester may be formed with any organic or inorganic acid. Suitably for the purpose of the present invention, appropriate acids for foods, drinks, pharmaceuticals or cosmetics may be used. Preferably, fatty acids may be used. Examples of the fatty acids may include, but not be limited to, long chain fatty acids such as oleic acid, palmitic acid, stearic acid, linoleic acid or linoleinic acid; and short or medium- chain fatty acids such as butyric acid or acetic acid.

The salts of the above compounds may also suitably be used for the present invention. The solution of the compound may be formed by mixing with appropriate acids acceptable for foods, drinks, pharmaceuticals or cosmetics such as hydrochloric acid, sulfuric acid, methanesulfonic acid, fumaric acid, maleic acid, succinic acid, acetic acid, benzoic acid, oxalic acid, citric acid, tartaricacid, carbonic acid or phosphoric acid. Also, where the compound has an acidic moiety, an appropriate salt acceptable thereof for foods, drinks, pharmaceuticals or cosmetics may be used. Examples of the salt include the salt formed with an alkaline metal salt such as sodium, potassium salt; an alkaline earth metal salt such as calcium or magnesium salt; and a suitable organic ligand such as a quaternary ammonium salt.

The glycosides bound to the sugar moiety through one or more hydroxyl group may suitably be used in the present invention. The sugar moiety may be any sugar. The sugar moiety may include but not be limited to monosaccharides, disaccharides, trisaccharides, oligosaccharides or polysaccharides.

Where the compound has at least one asymmetric center, it may exist in an enantiomer form. Where the compound has at least two asymmetric centers, it may exit in a diastereomer form. Such an isomer and a mixture at any ratio thereof may be encompassed by the scope of the invention. The compounds may optionally be substituted.

In the present invention, the process for preparing glabrene, glabridin, glabrol, 3'-hydroxy-4'-*O*-methylglabridin, 4' -*O*-methylglabridin and hyspaglabridin B is not particularly limited, and these compounds can be obtained from G. *glabra* licorice. When the compounds are obtained from the licorice, the method is not particularly limited, and the compounds are contained in a hydrophobic licorice extract obtained by extraction with an organic solvent such as ethanol, ethyl acetate and acetone, and the extract may be used as it is, but a crude or purified product obtained by purifying the hydrophobic extract by column treatment, deodorization treatment, decolorization treatment etc. may also be used. As a matter of course, the compounds used in the present invention may be compounds derived from natural materials such as other plants, chemically synthesized compounds, or compounds biosynthesized in cultured cells. The compounds used may be purified compounds, but the compounds purified in a crude form can also be used insofar as they do not contain impurities unsuitable as foods, drinks, pharmaceutical preparations, non-pharmaceutical preparations, or cosmetics etc.

The fats and oils used in the present invention are glycerin fatty acid esters containing medium-chain fatty acid triglycerides, preferably the glycerin fatty acid esters containing at least about 50 wt% medium-chain fatty acid triglycerides, more preferably the glycerin fatty acid esters containing at least about 70 wt% medium-chain fatty acid triglycerides. The medium-chain fatty acid triglycerides comprise about C6 to C12 fatty acids as constituent fatty acids wherein the proportion of constituent fatty acids is not particularly limited, but the proportion of constituent of about C8 to C10 fatty acids is preferably at least about 50 wt% or more, more preferably at least about 70 wt% or more. In particular, medium-chain fatty acid triglycerides having a specific gravity of about 0.94 to 0.96 at about 20°C and a viscosity of about 23 to 28 cP at about 20°C are more preferable. The medium-chain fatty acid triglycerides used may be those derived from natural materials or prepared by ester exchange.

The fats and oils used in the present invention are the glycerin fatty acid esters containing partial glycerides, preferably glycerin fatty acid esters containing at least about 50 wt% or more partial glycerides, more preferably glycerin fatty acid esters containing at least about 70 wt% or more partial glycerides. The partial glycerides are diglycerides (1,2-diacylglycerol, 1,3-diacylglycerol) or monoglycerides (1-monoacylglycerol, 2-monoacylglycerol), and the diglycerides and/or the monoglycerides may be used, but from the viewpoint of processability, the diglycerides are preferable. The partial glycerides used may be those derived from natural materials or prepared by ester exchange.

As the fats and oils used in the present invention, a mixture of the medium-chain fatty acid triglycerides and partial glycerides may be used, or partial glycerides of medium-chain fatty acids may be used.

In the present invention, the method of dissolving glabrene, glabridin, glabrol, 3'-hydroxy-4'-*O*-methylglabridin, 4'-*O*-methylglabridin and hyspaglabridin B in fats and oils is not particularly limited, and can be carried out by a usual stirring or mixing procedure. When an extract containing the compounds or a crude preparation of the compounds is used, impurities other than the compounds are contained therein, and thus the compounds are dissolved in fats and oils by a stirring or mixing procedure, and then impurities insoluble in the fats and oils are desirably removed by a procedure such as filtration or centrifugation. When a purified preparation of the compounds is used, a solution can easily be obtained. When an extract containing the compounds or a crude preparation of the compounds is used, it is dissolved in an organic solvent such as ethanol, and then the resulting solution is mixed with fats and oils, and the organic solvent can be distilled away.

Generally, glabrene, glabridin, glabrol, 3'-hydroxy-4'-*O*-methylglabridin, 4'-*O*-methylglabridin and hyspaglabridin B are unstable in a powdery state, and the stability of these compounds cannot be improved even by dissolving them in an organic solvent such as ethanol. However, the stability of the compounds can be improved by dissolving them in the fats and oils used in the present invention. Further, the compounds are sparingly soluble in water, and the absorptivity for said compounds can be improved by dissolving them in the fats and oils used in the present invention.

The composition of the present invention may comprise a vehicle acceptable for foods, drinks, pharmaceuticals or cosmetics. The vehicle may be for example any inactive, organic or inorganic material suitable for oral, enteral, transdermal, subcutaneous or parenteral administration. Examples of the vehicle may include, but not limited to, water, gelatin, acasia, lactose, microcrystalline cellulose, starch, sodium starch glycorate, potassium hydrogen phosphate, magnesium stearate, talc, and colloidal silicon dioxide, or the like. Such a composition may also comprise other pharmacological agents and conventional additives such as stabilizers, wetting agents, emulsifying agents, flavors, and buffers, or the like.

The fat and oil processed composition for prevention and/or amelioration of life-style related disease and/or the fat and oil processed composition for inhibition and/or amelioration of increase in body weight according to the present invention can be utilized in a food, a drink such as health food and a food with health claims (a food for specified uses [FOSHU] and a food with nutrient function claims) , pharmaceutical preparations, non-pharmaceutical preparations, cosmetics etc. without any particular limit to their form. For example, the fats and oils having at least one compound selected from the group consisting of glabrene, glabridin, glabrol, 3'-hydroxy-4' -*O*-methylglabridin, 4' -*O*-methylglabridin and hyspaglabridin B dissolved therein can be used alone for cooking, soft capsules and lotion. Further, they are freely miscible with oily moieties, and can thus be mixed with other fats and oils to regulate their physical properties, depending on the object. In this case, the other fats and oils are preferably foods or pharmaceutical preparations, and their type and amount are determined in consideration of physical properties necessary for individual products or various conditions such as the temperature range in which they are used, and characteristics such as viscosity and melting point can be controlled by adjusting their type and amount. For example, vegetable oils such as corn oil, rapeseed oil, high erucic rapeseed oil, soybean oil, olive oil, safflower oil, cottonseed oil, sunflower oil, rice bran oil, palm oil and palm kernel oil, animal oils such as fish oil, tallow, lard, milk fat, and yolk oil, or fats and oils obtained by fractionation, hydrogenation or ester exchange of the above oils as the starting material, or mixed oils thereof can be used.

The fat and oil processed composition obtained in the manner described above can be used as liquid fats and oils such as salad oil and fry oil and as plastic fats and oils such as margarine and shortening, and can be used in water-in-oil emulsion and oil-in-water emulsion. The fat and oil processed composition may be converted by spray drying into powder usable as powdery fats and oils. The fat and oil processed composition for foods, drinks, produced from such fats and oils as the starting material, includes supplements of various types, such as soft capsules, hard capsules, tablets, chewable, bars and sachets, confectionery such as chewing gum, chocolates, candies, jellies, biscuits and crackers, frozen sweets such as ice cream and ice, drinks such as lactic drinks, soft drinks, vitamin drinks and beauty drinks, noodles such as udon (thick white noodles) , Chinese noodles, spaghetti and instant Chinese noodles, products made with boiled fish paste, such as kamaboko (boiled fish sausage) , chikuwa (tubular roll of boiled grilled fish paste) and hanpen (light, puffy cake made of ground fish), and seasonings such as dressing, mayonnaise and Worcester sauce, as well as bread, ham, soup, various kinds of retort-packed food and various kinds of frozen food, and the fat and oil processed composition for foods and drinks can also be used in pet foods and feeds for domestic animals.

For the purpose of nutrition enrichment, various vitamins such as vitamins A, C, D and E may be added for simultaneous use, or tasting agents such as various salts, spices, milk-related materials such as whole fat powdered milk, powdered skimmilk, fermented milk and butterfat may be added for simultaneous use. As starting materials other than those described above, any antioxidants and colorants used in usual oil-in-water emulsion and water-in-oil emulsion can be used.

When the fat and oil processed composition is used as soft capsules formulation, at least one compound selected from the group consisting of glabrene, glabridin, glabrol, 3'-hydroxy-4' -*O*-methylglabridin, 4' -*O*-methylglabridin and hyspaglabridin B is contained in a total amount of about 0.01 to 150 mg, preferably about 0.1 to 150 mg, more preferably about 1 to 50mg, per capsule. Depending on the purpose, various vitamins such as vitamins A, C, D and E, base materials for preparations such as beeswax, and an emulsifier may be further added. In this case, the emulsifier includes, but is not limited to, glycerin fatty acid esters, sucrose fatty acid esters, sorbitan fatty acid esters, polysorbates, and phospholipids. Among these compounds, glycerin fatty acid esters are preferable, and polyglycerin fatty acid esters are more preferable. The polyglycerin fatty acid esters particularly preferably are diglycerin monooleate or polyglycerin-condensed ricinoleate.

Glabrene, glabridin, glabrol, 3'-hydroxy-4'-*O*-methylglabridin, 4'-*O*-methylglabridin and hyspaglabridin B, contained in the fat and oil processed compositions for prevention and/or amelioration of life-style related disease and/or the fat and oil processed composition for inhibition and/or amelioration of increase in body weight according to the present invention are contained in the fat and oil processed composition desirably in such an amount as to permit the compounds to be ingested as the total amount in an amount of about 0.01 to 10 mg/kg (body weight), preferably about 0.1 to 1 mg/kg, per adult a day, in order to exhibit their effect on prevention and/or amelioration of life-style related diseases and/or on inhibition and/or amelioration of increase in body weight.

However, although the effective amount may vary depending on the age, body weight, health condition, and severity of the condition of the subject, mode and time of intake, excretion ratio of the mammal subject, or combination of the composition, it can experimentally or empirically be determined by the method known by those skilled in the art. The present invention provides a method for prevention and/or amelioration of life- style related diseases comprising administrating to a mammal subject in need or needless thereof the composition of the present invention. The amelioration may be estimation by the subject him/herself or a healthcare worker, and may be subjective (e. g. opinion) or objective (e.g. measurable by test or diagnostic methods).

### Examples

Hereinafter, the present invention is described in more detail by reference to the Examples, but the present invention is not limited to the Examples.

### Example 1

Licorice *(G. glabra)* harvested in Afghanistan, 9.85 kg, was subjected to extraction with ethanol (extracted twice with 49.25 L at 45°C for 2 hours) and then the resulting extract was concentrated to give 4.4 L concentrate. Further, 3 L of the concentrate was concentrated, then treated with activated charcoal and concentrated to give 811.2 g of ethanol solution containing a hydrophobic licorice extract. The rest (1.4 L) of the concentrate was used in Comparative Example 1.

The above ethanol solution, 629.2 g (containing 125.8 g of a hydrophobic licorice extract), was mixed with 187.6 g of Actar M-2 (medium-chain fatty acid triglycerides [MCT] having a fatty acid composition of C8 : C10 = 99 : 1, manufactured by Riken Vitamin), and the mixture was kept at about 80°C under stirring for about 1 hour, and then concentrated under reduced pressure to remove the ethanol. The concentrate was filtered with suction to separate insolubles, and after the filtration, 45.7 g of MCT was further added to the filtrate to give 297.0 g of MCT solution as a fat and oil processed composition.

The resulting MCT solution, 1 g, was dissolved in methanol for HPLC so as to give 100 ml in total. This solution was analyzed as a sample by HPLC under the following conditions, and as a result, glabrene, glabridin, glabrol and 4' -*O*-methylglabridin were contained in amounts of 6.6 mg, 30.8 mg, 12.6 mg and 3.7 mg, respectively, per g of the MCT solution (total amount, 53.7 mg; content, 5.4% by weight).

### HPLC analysis conditions

An analytical column J'sphere ODS-H80, 4.6x250 mm (YMC) was used at a column temperature of 40°C. As the mobile phase, a mixture of 20 mM aqueous phosphoric acid and acetonitrile was used at a flow rate of 1 ml/min. under a gradient condition where the concentration of acetonitrile was kept at 35% over a period of 20 min. after the start of analysis, then increased linearly to 70% over a period of from 20 min. to 75 min., and increased to 80% over a period of from 75 min. to 80 min. The injection volume was 20 µl, and the detection wavelength was 254 nm. The retention time of each compound was 40.0 min. for glabrene, 50.2 min. for glabridin, 58.3 min. for glabrol and 66.8 min. for 4'-*O*-methylglabridin.

### Comparative Example 1

The concentrate used in Example 1 was evaporated to dryness and ground to give a hydrophobic licorice extract, then 100 mg of which was then dissolved in methanol for HPLC so as to give 100 ml in total. This solution was analyzed as a sample by HPLC, and as a result, glabrene, glabridin, glabrol and 4' -*O*-methylglabridin were contained in amounts of 16.5 mg, 90.4 mg, 34.1 mg and 9.3 mg, respectively, per g of the hydrophobic licorice extract.

### Comparative Example 2

The ethanol solution used in Example 1, 100mg, was dissolved in methanol for HPLC to give 20 ml solution. This solution was analyzed as a sample by HPLC, and as a result, glabrene, glabridin, glabrol and 4' -*O*-methylglabridin were contained in amounts of 3.4 mg, 18.7 mg, 7.1 mg and 1.9 mg, respectively, per g of the ethanol solution.

### Shelf stability test

A shelf stability test of glabrene, glabridin, glabrol and 4'-*O*-methylglabridin in the above MCT solution was carried out as an accelerating test at 80°C. The MCT solution was kept at 80°C in a water bath, and the contents of glabrene, glabridin, glabrol and 4' -*O*-methylglabridin were determined after storage for 1, 3 and 5 hours respectively. The shelf stability was expressed in terms of the ratio of the content after storage to the content before storage, assuming that the content of each compound before storage was 100%.

The results of the shelf stability test are shown in Table 1. In the table, Compound 1 refers to glabrene, Compound 2 to glabridin, Compound 3 to glabrol, and Compound 4 to 4'-*O*-methylglabridin.

A shelf stability test of the hydrophobic licorice extract in Comparative Example 1 was carried out in an analogous manner. The results are shown in Table 2.

A shelf stability test of the ethanol solution in Comparative Example 2 was carried out in an analogous manner. The results are shown in Table 3.

**Table 1**

| MCT solution | Shelf stability | | | |
|---|---|---|---|---|
| | Compound 1 | Compound 2 | Compound 3 | Compound 4 |
| At the time of start | 100% | 100% | 100% | 100% 100% |
| After 1 hour | 100% | 100% | 101% | 97% |
| After 3 hours | 99% | 101% | 103% | 103% |
| After 5 hours | 97% | 97% | 99% | 97% |

**Table 2**

| Hydrophobic licorice extract | Shelf stability | | | |
|---|---|---|---|---|
| | Compound 1 | Compound 2 | Compound 3 | Compound 4 |
| At the time of start | 100% | 100% | 100% | 100% |
| After 1 hour | 91% | 91% | 97% | 92% |
| After 3 hours | 88% | 88% | 101% | 98% |
| After 5 hours | 85% | 86% | 103% | 99% |

**Table 3**

| Ethanol solution | Shelf stability | | | |
|---|---|---|---|---|
| | Compound 1 | Compound 2 | Compound 3 | Compound 4 |
| At the time of start | 100% | 100% | 100% | 100% |
| After 1 hour | 94% | 96% | 95% | 89% |
| After 3 hour | 93% | 94% | 95% | 93% |
| After 5 hour | 91% | 93% | 95% | 90% |

Compounds 1 to 4 were stable in the MCT solution, were stable, while in the hydrophobic licorice extract and the ethanol solution, Compounds 1 and 2 in particular were unstable and their contents were reduced with time during storage.

From these results, it was revealed that Compounds 1 and 2 are unstable in the hydrophobic licorice extract and the ethanol solution, while Compounds 1 to 4 are stable in the MCT solution. This test is an accelerating test at 80°C, and thus it was revealed that they are stable for several days to several months under the condition of room temperature or thereabout.

### Example 2

From 103.7 g of ethanol solution containing 51.9 g of hydrophobic licorice extract and 468.2 g of MCT, 497.4 g of MCT solution was obtained in the same manner as that of Example 1. This MCT solution was analyzed by HPLC, and as a result, glabrene, glabridin, glabrol and 4' -*O*-methylglabridin were contained in amounts of 1.2 mg, 12.3 mg, 1.9 mg and 0.9 mg, respectively, per g of the MCT solution (total amount, 16.3 mg; content, 1.6% by weight).

### Example 3

### Action on reduction in visceral fat and inhibitory effect on increase in body weight

C57BL/6Jmice (female, 8-week-old) were allowed high fat/high sugar diet *ad libitum* for 8 weeks to cause diet-induced obesity. The high fat/high sugar diet used was semisolid purified feed composed of 25 wt% casein, 14.869 wt% corn starch, 20 wt% sucrose, 2 wt% soybean oil, 14 wt% lard, 14 wt% beef tallow, 5 wt% cellulose powder, 3.5 wt% AIN-93 mineral mixture, 1 wt% AIN-93 vitamin mixture, 0.25 wt% choline bitartrate, 0.006 wt% *tert-*butylhydroquinone and 0.375 wt% L-cystine (Oriental Yeast Co., Ltd.).
Thereafter, the mice were divided into 4 groups (groups A, B, C and D, each consisting of 10 mice), and powdery high fat/high sugar diet (having the same components as described above in a powdery state) to which the MCT solution in Example 2 and MCT had been added as shown in Table 4 was given to each group *ad libitum* for 8 weeks. As MCT, Actar M-2 (Riken Vitamin) was used.

After administration of the sample, the peritoneal cavity of each mouse under ether anesthesia was opened, and after the mouse was killed by collecting blood through an abdominal aorta, mesenteric fat, perirenal fat and periuterine fat removed and then weighed. The total weight of the mesenteric fat, perirenal fat and periuterine fat was regarded as the weight of intra-abdominal fat. A change in weight of each group, and a change in the measured weight of fat, are shown in Table 5.

There was no recognized difference in the amount of ingested feed among the respective groups, but a significant inhibitory effect on increase in body weight was recognized in groups B to D as compared with group A. Significant reduction in weights of mesenteric fat, perirenal fat, periuterine fat and intra-abdominal fat was also recognized in groups B to D as compared with group A. That is, it was revealed that when the mice which have developed diet-induced obesity with high fat/high diet are allowed the food to which at least 1% MCT solution in Example 2 was added, there are a significant inhibitory effect on increase in body weight and a significant inhibitory effect on accumulation of intra-abdominal fat.

From this result, it was revealed that the MCT solution containing glabrene, glabridin, glabrol, 3'-hydroxy-4'-*O*-methylglabridin, 4'-*O*-methylglabridin and hyspaglabridin B is effective in inhibiting the increase of body weight and in reducing visceral fat under the condition of excessive nutrition by ingestion of high fat/high sugar food.

**Table 4**

| Group | Amount | |
|---|---|---|
| | MCT solution | MCT |
| Group A | - | 3 wt% |
| Group B | 1 wt% | 2 wt% |
| Group C | 2 wt% | 1 wt% |
| Group D | 3 wt% | - |

**Table 5**

| | Group A | Group B | Group C | Group D |
|---|---|---|---|---|
| Body weight (g) | | | | |
| At the time of start | 27.8 ± 3.2 | 27.6 ± 2.9 | 27.7 ± 2.9 | 27.5 ± 2.8 |
| After 8 weeks | 31.7 ± 4.1 | 27.2 ± 3.7* | 25.8 ± 2.3** | 24.6 ± 1.2** |
| Average amount of ingested feed (g/day/mouse) | 2.15 | 2.21 | 2.08 | 1.95 |

| Amount of fat (g) | | | | |
|---|---|---|---|---|
| - Mesenteric fat Mesenteric fat | 0.615 | 0.306 | 0.238 | 0.184 |
| | ± 0.184 | ± 0.109* | ± 0.093** | ± 0.049** |
| - Perirenal fat -Personal fat | 0.912 | 0.446 | 0.305 | 0.186 |
| | ± 0.433 | ± 0.207 | ± 0.181** | ± 0.076** |
| - Periuterine fat -Periuterine | 1.279 | 0.670 | 0.457 | 0.307 |
| | fat 0.437 | ± 0.353 | ± 0.205** | ± 0.131** |
| - Intra-abdominal fat | 2.806 | 1.421 | 1.000 | 0.667 |
| | -Intra-abdominal fat | ± 0.647 | ± 0.461** | ± 0.238** |

| | | | | |
|---|---|---|---|---|
| Mean ± SE (n = 10), * (P < 0.05) ** (p < 0.01) | | | | |

### Example 4

### Production of soft capsules (1)

The MCT solution in Example 1 was injected into a gelatin film by a rotary soft capsule production unit to give a soft capsule with a content of 350 mg. Glabrene, glabridin, glabrol and 4' -*O*-methylglabridin were contained in amounts of 2.3 mg, 10.8 mg, 4.4 mg and 1.3 mg, respectively, per capsule (total amount, 18.8 mg; content, 5.4% by weight).

### Production of soft capsules (2)

In 57 parts by weight of MCT(Actar M-2, Riken Vitamin Co. Ltd.), 3 parts by weight of diglycerin monooleate (DO-100V, Riken Vitamin) were dissolved in 57 parts by weight of MCT (Acter M-2, Riken Vitamin) , and 40 parts by weight of the MCT solution in Example 1 were added thereto under stirring to prepare a uniform solution.

This solution was injected into a gelatin film by a rotary soft capsule production unit to give a soft capsule with a content of 350 mg. Glabrene, glabridin, glabrol and 4'-*O*-methylglabridin were contained in amounts of 0. 9 mg, 4.3 mg, 1. 8 mg and 0. 5 mg, respectively, per capsule (total amount, 7.5 mg; content, 2.1% by weight).

### Example 5

### Production of powdery fat and oil

In 400 parts by weight of water, 70 parts by weight of dextrin and 20 parts by weight of Na casein were dissolved to prepare an aqueous phase.

To the prepared aqueous phase, 10 parts by weight of the MCT solution in Example 1 were added under stirring and then emulsified with a homogenizer, and the resulting emulsion was spray-dried to give powdery fat and oil.

### Example 6

### Production of margarine

To 80 parts by weight of hardened cottonseed oil (trade name: Snow Light, Kanegafuchi Chemical Industry Co., Ltd.), 20 parts by weight of the MCT solution in Example 1 and 10 parts by weight of salt-free butter (Yotsuba Nyugyo Co. , Ltd. ) , 0. 2 part by weight of glycerinmonofatty acid ester (trade name: Emulgy MS, Riken Vitamin) and 0.2 part by weight of lecithin were added, and then dissolved by heating at 60°C to prepare an oil phase.

To 84.9 parts by weight of the prepared oil phase, 15.1 parts by weight of water were added under stirring, then emulsified for 20 minutes, thereafter, cooled and kneaded by a combinator to prepare margarine.

### Example 7

### Production of conc. milk

After 10 parts by weight of the MCT solution in Example 1 were heated to 70°C, and then 0.1 part by weight of lecithin and 0.1 part by weight of polyglycerin fatty acid ester were successively dissolved therein to prepare an oil phase.

Separately, 25 parts by weight of powdered skin milk, 0.1 part by weight of glycerin fatty acid ester and 0.1 part by weight of sucrose fatty acid ester were dissolved in 64.6 parts by weight of water at 60°C to prepare an aqueous phase.

The prepared aqueous and oil phases were preliminarily emulsified and then sterilized at 145°C for 4 seconds with a UHT sterilizer. Then, the emulsion was cooled under vacuum and homogenized at a pressure of 10 MPa with a homogenizer and then cooled to 10°C with a plate to give processing conc. milk.

### Example 8

### Production of mayonnaise

Into a mixer, 10 parts by weight of vinegar, 1 part by weight of table salt, 0.6 part by weight of sugar, 0.2 part by weight of mustard powder and 0.2 part by weight of sodium glutamate were introduced, and then stirred and mixed at 15 to 20°C to prepare an aqueous phase. Thereafter, an emulsion (10 to 15°C) prepared by adding 10 parts by weight of yolk to 68 parts by weight of rice white squeezed oil and 10 parts by weight of the MCT solution in Example 1 and then emulsifying the mixture under stirring was added little by little to the above aqueous phase under stirring at 15 to 20°C to give a preliminarily emulsified mixture. Then, the mixture was emulsified with a colloid mill to give mayonnaise.

### Industrial Applicability

According to the present invention, there can be provided a fat and oil processed composition for prevention and/or amelioration of life-style related disease and/or a fat and oil processed composition for inhibition and/or amelioration of increase in body weight, which can be utilized in foods and drinks such as foods with health claims (foods for specified health uses [ FOSHU] and foods with nutrient function claims) , pharmaceutical preparations, non-pharmaceutical preparations, cosmetics etc. The fat and oil processed composition of the invention is effective in amelioration of insulin resistance, in prevention and/or amelioration of life-style related diseases such as visceral fat obesity, type 2 diabetes, hyperlipemia and hypertension, and/or in inhibition and/or amelioration of obesity i.e. increase in body weight.

## Claims

1. A fat and oil processed composition for prevention and/or amelioration of a life-style related disease, in which at least one compound selected from the group consisting of glabrene, glabridin, glabrol, 3'-hydroxy-4'-O-methylglabridin,4' -*O*-methylglabridin and hyspaglabridin B or a salt, an ester or a glycoside thereof is dissolved in the fat and oil, wherein the total amount of the compounds is about 0.01 to 30% by weight based on the composition.

2. A fat and oil processed composition for inhibition and/or amelioration of increase in body weight, in which at least one compound selected from the group consisting of glabrene, glabridin, glabrol, 3'-hydroxy-4'-O-methylglabridin, 4' -*O*-methylglabridin and hyspaglabridin B or a salt, an ester or a glycoside thereof is dissolved in the fat and oil, wherein the total amount of the compounds is about 0.01 to 30% by weight based on the composition.

3. A fat and oil processed composition for amelioration of insulin resistance, in which at least one compound selected from the group consisting of glabrene, glabridin, glabrol, 3'-hydroxy-4'-O-methylglabridin, 4'-*O*-methylglabridin and hyspaglabridin B or a salt, an ester or a glycoside thereof is dissolved in the fat and oil, wherein the total amount of the compounds is about 0.01 to 30% by weight based on the composition.

4. The fat and oil processed composition according to any one of claims 1 to 3, in which at least one compound selected from the group consisting of glabrene, glabridin, glabrol, 3'-hydroxy-4' -*O*-methylglabridin, 4' -*O*-methylglabridin and hyspaglabridin B or a salt, an ester or a glycoside thereof is contained in such an amount that about 0.01 to 10 mg/kg body weight per adult a day may be ingested in total of the compounds.

5. The fat and oil processed composition according to any one of claims 1 to 4, wherein the fat or oil comprises a glycerin fatty acid ester containing a medium-chain fatty acid triglyceride and/or a partial glyceride.

6. The fat and oil processed composition according to any one of claims 1 to 5, wherein the fat or oil comprises a glycerin fatty acid ester containing a medium-chain fatty acid triglyceride in an amount of about 50 % by weight or more.

7. The fat and oil processed composition according to any one of claims 1 to 5, wherein the fat or oil comprises a glycerin fatty acid ester containing a partial glyceride in an amount of about 50 % by weight or more.

8. The fat and oil processed composition according to claim 1, wherein the life-style related disease comprises at least one member selected from the group consisting of visceral fat obesity, type 2 diabetes, hyperlipemia, and hypertension.

9. The fat and oil processed composition according to claim 1, wherein the life-style related disease is visceral fat obesity and/or type 2 diabetes.

10. The fat and oil processed composition according to any one of claims 1 to 9, which is used for a food or a drink.

11. The fat and oil processed composition according to any one of claims 1 to 9, which is used for a pharmaceutical preparation.

12. The fat and oil processed composition according to any one of claims 1 to 9, which is used for a cosmetic.

13. A soft capsule formulation comprising the fat and oil processed composition according to any one of claims 1 to 12 encapsulated therein.

14. The soft capsule formulation according to claim 13, wherein the capsule comprises at least one compound selected from the group consisting of glabrene, glabridin, glabrol, 3'-hydroxy-4'-*O*-methylglabridin, 4'-*O*-methylglabridin and hyspaglabridin B or a salt, an ester or a glycoside thereof in an total amount of about 0.01 to 150 mg per capsule.

15. The fat and oil processed composition according to any one of claims 1 to 14, wherein at least one compound selected from the group consisting of glabrene, glabridin, glabrol, 3'-hydroxy-4'-*O*-methylglabridin, 4'-*O*-methylglabridin and hyspaglabridin B or a salt, an ester or a glycoside thereof is obtainable from *Glycyrrhiza glabra.*

16. A process for producing the composition according to any one of claims 1 to 15, which comprises dissolving at least one compound selected from the group consisting of glabrene, glabridin, glabrol, 3'-hydroxy-4' -*O*-methylglabridin, 4' -*O*-methylglabridin and hyspaglabridin B or a salt, an ester or a glycoside thereof in a fat or an oil.

17. A method for prevention and/or amelioration of a life-style related disease, which comprises administering an effective amount of the composition according to any one of claims 1 to 15 to a mammal subject in need thereof.
